# EUROPEAN PATENT APPLICATION

(11) **EP 3 613 417 A1**
(43) Date of publication of application: **26.02.2020**
(21) Application number: 18787606.5
(22) Date of filing: 20.04.2018
(51) Int. Cl.: A61K 31/185, A61K 33/04, A61P 35/00, A61P 31/00

(54) **METHOD FOR INHIBITING CELLULAR GLYCOLYSIS PROCESS AND APPLICATION THEREOF**

(30) Priority: 22.04.2017 CN 201710285989; 02.05.2017 CN 201710324594; 17.08.2017 CN 201710740788; 27.09.2017 CN 201710956579
(71) Applicant: Qin, Caidong, Anhui 243000 (CN)
(72) Inventor: Qin, Caidong, Anhui 243000 (CN)
(74) Representative: Pfenning, Meinig & Partner mbB
(86) International application number: PCT/CN2018/083952
(87) International publication number: WO 2018/192573

(57) **Abstract**

A method for inhibiting a cellular glycolysis process and an application thereof. The method is using reductive selenium dioxide, selenite or formic acid, formate, oxalic acid, oxalate, sulfur-containing reducing agent, and the like to inhibit a hydrogen cyclic process required in a redox reaction in the cellular glycolysis process. The application comprises : tumor prevention and treatment by applying different reducing agents by means of injection, oral administration, external application, spraying, or fumigation; prevention and treatment of digestive tract inflammation and tumors by means of oral administration; inhibition of a metabolic process of anaerobes and facultative anaerobes, where the reductive selenium dioxide, selenite, formic acid, formate, and the like are used as one of the active ingredients or ingredients of a fungicide; and disease prevention and antioxidation of fresh goods, food, fish and meat, vegetables and fruits, natural perfume, natural cosmetics, detergents, skin care products, and daily sanitary products, maintaining of human redox equilibrium, preservation and fresh-keeping, or related health care field. Problem caused by acidity of the body fluid can be solved by using alkaline sodium carbonate, which is the product of sodium formate and sodium oxalate after metabolism.

## Description

### Technical Field

The present invention relates to a method for inhibiting the glycolysis process in cells and use thereof, pertaining to the technical fields of prevention and treatment of tumors and microbial/bacterial infections, and prevention of corruption caused by microbes/bacteria, including its applications in antisepsis/preservatives for beverages, foods, cosmetics made from natural raw materials and natural perfume, as well as detergents, skin care products, sanitation, medical care, the human body redox equilibrium and acid-base balance.

### Background Art

It is well known that tumor cells or cells of anaerobes or facultative anaerobes gain energy by aerobic or anaerobic glycolysis, whereas normal cells gain energy by aerobic oxidation. Glycolysis refers to a process of decomposing glucose or glycogen into pyruvate, ATP and NADH+H⁺. The formation of ATP and NADH will inevitably lead to a decrease in the substrates NAD⁺ and ADP, which in turn will severely suppress the progress of glycolysis. ATP is rapidly consumed *in vivo* to produce ADP and phosphate, and thus the inhibitory effect of ATP is almost negligible. NADH is oxidized to give water in mitochondria in the presence of oxygen to reproduce NAD⁺, but this process cannot proceed in cells without mitochondria or in the absence of oxygen. Hence a decrease in NAD⁺ and an increase in NADH have a very significant inhibitory effect on glycolysis in cells without mitochondria or oxygen.

The aerobic oxidation and glycolysis of sugars are the same in the initial stage, but differ from each other after decomposition into pyruvate due to different conditions of oxygen supply or different manners of oxygen utilization.

Each step of glycolytic chemical reaction is catalyzed by a corresponding enzyme. The total reaction is expressed as follows:

Glucose + 2ATP + 2ADP + 2Pi + 2NAD⁺ → 2 pyruvate + 4ATP + 2NADH + 2H⁺

Pyruvate (CH₃COCOOH) + NADH + H⁺ ←(reversible)→ lactate (CH₃CHOHCOOH) + NAD⁺

Specifically, two important redox reactions are involved in the metabolism of sugar into lactate in tumor cells or anaerobe cells:

(1) Glyceraldehyde 3-phosphate + phosphate + NAD⁺ → 1,3-diphosphoglycerate + NADH + H⁺;

and

(2) Pyruvate + NADH + H⁺ → lactate + NAD⁺

In these reactions, NAD⁺ and NADH can be recycled and regenerated as a catalyst. Removal of the inhibitory effect of a NAD⁺ decrease and a NADH increase on glycolysis, that is, maintenance of the hydrogen transfer process required in cells through NAD⁺, is necessary to ensure the sustainability of the glycolysis process. The glycolysis process described herein includes a process in which the glycolysis product pyruvate is reduced to produce lactate, or a process in which pyruvate is decarboxylated by microorganisms to produce acetaldehyde which is then reduced by NADH to produce ethanol.

Although the steps of the metabolic chemical reactions in tumor cells and anaerobe cells have been known for many years, they have not been paid enough attention or successfully utilized by those skilled in the art, so that prevention and treatment of tumors, as a major issue to be solved urgent to all human beings, have been hampered by the limitations of the existing technology frameworks and modes of thinking, and still depend mainly on surgery, radiotherapy, chemotherapy, targeting drugs, and immunotherapy, without making breakthroughs into a novel therapeutic method like that in the present invention. There are also publicly reported therapies for preventing or treating cancerous tumors that involve traditional Chinese medicines or other chemicals, but none of them recognizes or contemplates that related substances may exert their functions or effects by influencing the steps of chemical reactions in metabolism in tumor cells.

### Summary of Invention

It is well known that reductive selenium and selenium compounds have a tumor-preventing effect, of which the mechanism has not yet been clearly elucidated and verified. It is generally believed that the anti-oxidizing selenium-containing substances neutralize free radicals in cells or complex toxic heavy metal ions to scavenge pathogenic factors, or that the anti-oxidizing selenium-containing substances take effect by covalently bonding to amino acids to form special selenocysteine and selenomethionine present in the living body, and this effect does not act on a specific chemical reaction in a cell, but is merely considered as a specific effect possessed by a specific substance, and thus cannot provide any guidance for treatment of a specific pathology, but can only illustrate and explain its preventive effect on tumors, including the preventive effect of selenium-containing amino acids or proteins on tumors. In the prior art, it has not been recognized or considered that chemical reductivity may produce a preventive and even direct therapeutic effect on tumors and inflammations by being directly or indirectly involved in and affecting the chemical reactions in cellular energy metabolism, which is essentially different from the anti-oxidative neutralizing effect on active substances such as free radicals.

An effect of prevention or treatment of tumors (including mutated cells in the blood) or killing and suppressing bacteria is often mentioned in the literature in summaries of properties of certain substances, but this effect was not explicitly attributed to the factor that the reducing powder of the substances is involved in or affects the cellular energy metabolism, let alone subjected to an innovative inductive reasoning by listing all such substances, just like the fact that the common characteristics of elements can only be found after the elements are arranged according to the periodic table. Those skilled in the art only considered this effect as an opportunistic characteristic specific to a specific form of an individual substance, rather than a common characteristic of such substances. For example, among all nitrogen oxides, only the typical reductive nitric oxide has an effect of treating tumors; reductive arsenic may be used to treat certain leukemia; reductive sodium selenite has an inhibitory effect on tumors; and reductive trivalent chromium can be used to treat tumors, but the oxidative hexavalent chromium is a strong carcinogen. These facts that have not been noticed and listed together to carry out a comparative and inductive reasoning are now listed and subjected to a comparative analysis according to the present invention, allowing the inventors to draw a theoretical conclusion and make a further experimental verification that: whether a simple compound has an effect of treating tumors or killing or suppressing bacteria lies in its reductivity, specifically, lies in that the reductivity directly participates in and affects intracellular metabolic chemical reactions to destroy the cycling between NAD⁺ and NADH which is unique in living tumor cells or living anaerobe cells, including the cycling between NADP⁺ and NADPH of glutamine in tumor cells. These are technical directions and phenomena that should have been studied but not yet noticed in the prior art due to a long-standing technical bias. This direct therapeutic effect on tumors has not been considered and summed up from the perspective of reductivity of substances, nor clearly attributed to reductivity. On the contrary, the reductivity of substances was only considered and valued when it overcomed the destructive effects of free radicals *in vivo* as an idea and concept of anti-oxidation, and was also valued in view of prevention of tumors. With respect to well-known concepts, a reducing agent must have reductivity and can be oxidized by an oxidizing agent, but an antioxidant generally does not necessarily have reductivity, but has high stability making it not easily oxidized by an oxidizing agent. Antioxidants are substances that prevent adverse effects of oxygen. Antioxidants in food can protect food from oxidative damage and deterioration. Antioxidants in the digestive tract of human prevent oxidative damage to the digestive tract, but they may not show a reducing chemical action in human cells.

In summary, acknowledge of the mechanism of action of specific forms of substances on human health, i.e. the theory that a specific form of a substance is responsible for a beneficial effect, for example, it was believed that the effect of reductive substances including VC, VE, and sodium selenite was due to their neutralization effect against excessive free radicals and harmful substances in the body, and that selenium may improve human immunity, promote proliferation of lymphocytes and synthesis of antibodies and immunoglobulins and synergistic anti-oxidation, and increase anti-oxidation activity, makes it difficult for persons skilled in the art to correct the long-standing technical bias. This technical bias that links therapeutic efficacy to a specific form and constitution of a substance limits the minds of those skilled in the art, and prevents any breakthrough in the field of cancer treatment and anaerobe sterilization.

Taking sodium selenite as an example, it was traditionally considered that selenium is known as an essential element of living organisms because it exists *in vivo* in the form of selenocysteine and selenomethionine formed by bonding with amino acids through covalent bonds. The former one is specifically involved in synthesis of proteins, while the latter one can non-specifically replace methionine in synthesis of proteins, playing a very important role in various synthesis and metabolism processes of life activities. In consideration of the reductivity of selenium according to the present invention, a method for inhibiting glycolysis in cells may be found based on an analysis of redox reactions involved in metabolic reactions of glycolysis in cells, and a relevant wide application thereof may be realized. In presence of sodium selenite (or other reducing agents), it is firstly proposed in the present invention that the reduction reaction of sodium selenite can be expressed by a chemical equation shown hereinafter, so that a mechanism by which various reducing agents can provide free hydrogen atoms through water in a cell can be understood, which is significant in that it breaks the stereotypical thought that the preventive effect on tumors is due to the intrinsic chemical properties of an individual specific substance such as "selenium" or the like, or the specific biochemical reactions involving the substance and macromolecules such as proteins or enzymes in tumor cells. Therefore, the present invention revolutionarily provides a directional guidance for use of a reducing agent as a drug. With this ground-breaking chemical equation, sodium selenite can be selected as an effective inhibitor against anaerobia, rather than just as an antioxidant for prevention of tumors, or as a supplement to the trace elements needed by a human body.

The reductant effect according to the present invention needs to solve the problem of how a hydrogen-free exogenous reducing agent (NADH and NADPH are endogenous reducing agents) can participate in the hydrogen cycle in the glycolytic chemical reactions. The reducing agent (including a reduction potential) is not directly involved in the metabolic chemical reactions in cells, but, indirectly, reacts with water first in the presence of water to release or preparatively release hydrogen atoms. These additional hydrogen atoms produced by the reducing agent break the concentration balance of reduced hydrogen atoms in cells and destroy the cycle of carrying and transferring hydrogen by hydrogen-carriers.

Na₂SeO₃ (highly reductive) + H₂O → Na₂SeO₄ (a stable compound) + 2H (atomic hydrogen)

(Other reducing agents may also undergo similar hydrogen-producing reactions.)

In addition, it is assumed that
with respect to the above reaction (1), sodium selenite serves as a reducing agent, and competes with the substrate glyceraldehyde 3-phosphate which is also a reducing agent, so the following redox reactions may occur respectively:

(a) sodium selenite + H₂O + NAD⁺ → NADH + H⁺ + sodium selenate

(b) glyceraldehyde 3-phosphate + phosphate + NAD⁺ → 1,3-diphosphoglycerate + NADH + H⁺

According to the change in free energy of the chemical reaction, it can be inferred that the driving energy for reaction (b) from aldehyde to acid should be greater than that for reaction (a). Therefore, reaction (1) is less affected by the presence of sodium selenite, thereby avoiding or reducing the interference from sodium selenite in the metabolism in normal cells. Alternatively, the reason may be that sodium selenite as a reducing agent competes with the substrate glyceraldehyde 3-phosphate which is also a reducing agent, but sodium selenite as a reducing agent is at an inferior position to participate in the enzyme-catalyzed specific reaction.

For above reaction (2), sodium selenite as a reducing agent directly competes with the coenzyme NADH which is also a reducing agent, rather than competes with the substrate pyruvate. If pyruvate accepts hydrogen provided by the reduction reaction between sodium selenite and water, NADH would not be converted into NAD⁺; alternatively, pyruvate may still accept hydrogen from NADH, converting NADH into NAD⁺, but NAD⁺ may immediately bond the atomic hydrogen provided by the reduction reaction between sodium selenite and water to form NADH. That is, the biochemical reaction in the cell catalyzes the above reaction of the reducing agent with water to provide hydrogen. Therefore, it can be expected that glycometabolism in tumor cells or in anaerobic bacterial cells would fail because NAD⁺ required by reaction (1) is unavailable in the glycolysis chain. The reducing agent hinders the hydrogen cycling process required by the redox reaction during glycolysis, and produces an effect of inhibiting growth of tumor cells and promoting apoptosis of tumor cells, and also inhibiting growth of anaerobe or facultative anaerobe cells and promoting apoptosis of bacterial cells. For microorganisms such as bacteria, the difference in the enzyme structure results in different specific products produced by the reduction of pyruvate. Alternatively, the presence of a reducing agent continuously increases the concentration of NADH, and the continuously increased reductivity changes the redox state of cells, causing tumor cells to die due to abnormal metabolism.

Normal cells are not affected by the combination of the reducing agent and water, that is, there is no toxic side effect on normal cells, because normal cells produce a large amount of NAD⁺ during oxidative phosphorylation. Even if NAD⁺ is partially consumed and converted into NADH by an exogenous reducing agent, there is still a large amount of NAD⁺ available for the dehydrogenation reaction, which ensures smooth proceeding of glycolysis, while the process of energy production from glutamine metabolism is not a main pathway for normal cells to obtain energy. Therefore, the combination of a reducing agent and water would hardly affect the energy-producing metabolism in normal cells, but only interfere with and end the energy-producing process in tumor cells.

It is reported in literature (Nature Communications: Transfer hydrogenation catalysis in cells as a new approach to anticancer drug design, Joan J. Soldevila-Barreda et al., Mar. 2015) that the combination of an organometallic complex as a chemotherapeutic catalyst and formate as a hydrogen donor can change the ratio between NAD⁺ and NADH, thereby achieving cancer treatment. However, this report points out that the organometallic complex or formate alone has no such catalytic effect. Unlike the present invention, this report incorrectly denies and excludes the efficacy of formate alone as a drug for treating tumors, probably because this report is a study on the *in vitro* catalytic conversion between NAD⁺ and NADH, wherein the catalytic effect of the organometallic complex replaces the induction catalytic effect of the biochemical reactions in tumor cells in a human body according to the present invention. Similarly, it is reported in literature (Cancer/Oncology: Stinging nettles: A new approach to cancer, Tim Newman, 11 Jan. 2018) that under the interaction with formate, an organometallic complex catalyst produces a special chiral molecule to change the growth mode of tumor cells, thereby killing the tumor cells. However, formate still only acts to induce the organometallic complex catalyst, rather than directly treats the tumor. This report also incorrectly denies and excludes potential of formate to be used as a drug and to directly treat tumors. These research results in the literature further illustrate that those skilled in the art still do not pay attention to or envisage a direct therapeutic effect of a reducing agent *per se* in the human body. Thus, the present invention innovatively utilizes the glycolytic chemical reaction steps in living cells in human bodies, and applies reducing agents to the treatment of tumors and bacterial infective diseases. It is also illustrated that, relying solely on the known glycolytic chemical reaction steps and principles in tumor cells, those skilled in the art cannot find by reasoning the therapeutic effect of a reducing agent or reductivity as a characteristic of a group of substances in two important areas: tumor treatment and microbial infection in humans.

In addition, since stem cells obtain energy mainly by glycolysis, a reducing agent can also inhibit growth of stem cells and promote their apoptosis. It is known that traditional methods for treating tumors, such as radiotherapy, chemotherapy, and targeted therapy, may result in tumor cells turning towards stem cells, and these turned tumor cells are an important mechanism or one of the important mechanisms of cancer recurrence and metastasis. Therefore, one of the objectives of the present invention is to use a reducing agent in combination with conventional therapeutic methods such as radiotherapy, chemotherapy and targeted therapy to help avoid tumor metastasis and recurrence.

Also as one of the objectives of the present invention, applicability and efficacy of existing reducing substances and the mechanism thereof are confirmed in the present invention, thereby providing a clear basis for its applications, and effectively expanding the scope of their specific applications to enable discovery of new drugs or new functions by converting substances that have never been drugs into drugs, rather than relying on random trials for efficacy and random combinations of different substances to find prescriptions. The latter cannot provide any effective and useful guidance for the application of reducing substances, which affects practical application and extension of the importance value of reductivity of these substances.

Therefore, one of the objectives of the present invention is, by controlling the chemical reductivity of substances, to maintain the redox balance in a human body, and to inhibit glycolysis in tumor cells and anaerobic respiration in microorganisms, thereby preventing and treating diseases associated with tumors and anaerobic microorganisms that mainly rely on aerobic glycolysis. In particular, the present invention solves the issue that reducing agents at a low concentration can only be used as an antioxidant for preventing tumor diseases but cannot be used as a therapeutic drug for tumor diseases. A high concentration refers to a level that has a high enough chemical potential according to the Nernst equation to affect the progress of redox reactions in the glycolytic metabolic process. A low concentration is due to the toxicity of the reducing agent. For example, for oral administration of sodium selenite, the publicly reported toxic dose for human is 400 mg per day, which limits the level of sodium selenite or selenium in human blood. However, by injection into the blood, under the premise of avoiding toxicity to the intestinal system, the same dose of 400 mg can effectively increase the blood concentration of sodium selenite, so that an inhibitory effect on the chemical reactions in tumor cells and thus a therapeutic effect can be produced. Therefore, the so-called high concentration means that the blood level of a reducing agent exceeds that achievable by conventional oral administration of the reducing agent at a safe oral dose.

It is confirmed in the present invention that reducing agents can disrupt the intrinsic redox state in tumor cells, and can block the energy metabolism reaction in tumors and microorganisms associated with glycolytic metabolic reactions. In practice, non-toxicity or low toxicity to the human body and reduced or few side effects by direct involvement in and interference with physiological metabolic reactions in normal cells are considered as well. Regarding the dosage, in order to fulfill the treatment purpose, it is necessary to achieve a drug concentration as high as possible, that is, as much as possible in a single dose, but with an upper-limit of just being non-toxic or low-toxic, to increase the use amount of the reducing agent while reducing the toxicity to normal cells and tissues as much as possible. When sulfur-containing reducing agents, such as sulfur-containing amino acids, sulfides (including but not limited to sulfur dioxide, hydrogen sulfide, sodium sulfide, α-thiopropionylglycine, lipoic acid, dihydrolipoic acid, thiol compounds, disulfide compounds, and the like), thiosulfate, sulfurous acid, sulfinic acid, sulfinate, dithionous acid, thiourea dioxide, and sulfite, are used, because of their strong reductivity or toxicity, they can only be used in a small amount by oral administration or injection, or in a small amount for oral administration or skin rubbing for prevention and treatment of tumors or anaerobic bacterial infections in the epidermis (including mucosal epidermis) and the digestive tract.

Another objective of the present invention is to solve various health problems caused by acidic body fluids, including suppressing excessive gastric acid and uric acid, by utilizing the characteristics that the metabolite of sodium formate, sodium oxalate or a corresponding potassium salt is alkaline sodium carbonate or potassium carbonate.

One of the objectives of the present invention is to develop a product to replace antibiotics, in the field of prevention and treatment of tumors and bacterial infections, and the field of prevention of putrefaction caused by bacteria or microorganisms, by utilizing a reducing agent's function of inhibiting glycolysis in tumor cells and bacterial cells.

In the field of tumor prevention, the corresponding products according to the present invention include: bottled water, tap water, beverage, wine, pickles, pickles in soy sauce, various food noodles, mouthwash, toothpaste, various orificial cleansers, skin-care products (including skin cleansers, soaps, masks, etc.), pills, spray, paste, fumigant, bath salt or the like that contains a small amount of the reducing agent.

In the field of tumor treatment, the corresponding products include: mouthwash, oral water aqua, oral pills, injections, pastes, spray, fumigants, bath salt or the like that contains a sufficient amount of the reducing agent.

In the field of prevention of bacterial infections, the corresponding products include mouthwash, toothpaste, various orificial and skin cleansers, soap, bath salt, masks, skin-care products, drinking water, bottled water or the like.

In the field of treatment of bacterial infections, the corresponding product include mouthwash, oral water aqua, oral pills, injections, pastes, cleansers, spray, fumigants, bath salt or the like that contains a sufficient amount of the reducing agent.

The method for inhibiting glycolysis in cells and the use thereof according to the present invention is a novel discovery of a function of existing reducing substances, in which reductive selenium dioxide, selenite (i.e., a selenium-containing reducing agent) or formic acid, formate, oxalic acid, oxalate, dioxalate, sulfinic acid, sulfinate, thiourea dioxide, reductive amino acids including but not limited to cysteine, cystine, methionine, and other sulfur-containing amino acids and its reductive derivatives (i.e., a carbon-containing reducing agent), thiocyanide (thiocyanate), thiosulfate, sulfide (hydrogen sulfide, sodium sulfide, etc.), sulfurous acid, sulfite, dithionous acid (i.e., a sulfur-containing reducing agent), metal nanoparticles, and nano-selenium particles are used to hinder the hydrogen cycling process required for the redox reactions in glycolysis, wherein the salts selenite, formate, oxalate, thiocyanate, thiosulfate, and sulfite include, but are not limited to, sodium salts, potassium salts, calcium salts, magnesium salts or ammonium salts. Although it is obvious that most of the reducing agents tend to be toxic to the human body, there must be a compromise made to maximize the overall benefit in the presence of a fatal tumor disease, just like many useful chemotherapy drugs despite their toxicity. Of course, it is preferable to use a reducing agent which is non-toxic and harmless.

In an embodiment of the method for inhibiting glycolysis in cells according to the present invention, a reducing substance including, but not limited to, formic acid, formate, oxalic acid, oxalate, dioxalate, sulfides (hydrogen sulfide, sodium sulfide, etc.), sulfurous acid, sulfite, sulfinic acid, sulfinate, dithionous acid, thiourea dioxide, thiocyanate, thiosulfate, reductive amino acids, metal nanoparticles (metal nanoparticles with low toxicity, such as nanosized silver, iron, molybdenum, manganese, chromium, vanadium, and low-valence reductive manganese or molybdenum compounds, etc.) is used, alone or in combination, in a form of mouthwash or by oral administration, for prevention or treatment of tumors. It is most preferably to use formate, oxalate, formic acid, and oxalic acid, which are non-toxic or less toxic and not directly involved in biochemical reactions in cells.

In an embodiment of the method for inhibiting glycolysis in cells according to the present invention, reductive selenium dioxide, selenite, formic acid, formate, oxalic acid, oxalate, dioxalate, sulfides (hydrogen sulfide, sodium sulfide, etc.), sulfurous acid, sulfite, sulfinic acid, sulfinate, dithionous acid, thiourea dioxide, thiocyanate, thiosulfate, reductive amino acids, metal nanoparticles (metal nanoparticles with low toxicity, such as nanosized silver, iron, molybdenum, manganese, chromium, vanadium, and low-valence reductive manganese or molybdenum compounds, etc.) and selenium nanoparticles are used, alone or in combination, in forms of transfusion by injection, paste for external application, spray, stifling agents, bath salt or mouthwash, for prevention or treatment of tumors. It is most preferably to use formate, oxalate, formic acid, oxalic acid and sodium selenite, which are non-toxic or less toxic.

The injection for transfusion aims to directly obtain a high blood concentration of a reducing agent, to solve the problem of a low concentration after absorption and transportation from the digestive tract into blood, including the problem of a limited effect due to the limited safe dose of selenium (no more than 400 mg per day) that limits the oral intake of the reducing agent. This problem may be the reason why the actual effect of current reducing selenium is compromised in treatment of tumors. The treatment effect of supplementation of selenium by oral administration on tumors were previously reported to vary from person to person, probably because the reductive selenium absorbed and transported from the digestive tract into blood have different concentrations in different persons. Therefore, one of the main objectives of the present invention is to overcome the toxicity problem of a reducing agent by screening and adopting a suitable administration path, and to use a high concentration of a reducing agent as a drug for treating tumors, so that a reducing agent, which is conventionally only a drug for preventing tumors (i.e., it can only act as an antioxidant), can also be used as a drug for treating tumors, rather than just as a substance or drug for preventing tumors or adjuvant treatment. By using a large dose of a non-toxic, harmless or low-toxic reducing agent as a drug for treating tumors and bacterial infections, the preventive effect of this agent as an antioxidant is expanded to a therapeutic effect of directly treating tumors and inhibiting and killing bacteria.

In an embodiment of the method for inhibiting glycolysis in cells according to the present invention, a low concentration of a sulfur-containing reducing agent, such as sulfinic acid, sulfinate, sodium thiosulfate, thiocyanate, dithionous acid, thiourea dioxide, sulfide (hydrogen sulfide, sodium sulfide, etc.), pastes of sulfur powder, nano-sulfur powder and sulfur fine powder, low-toxic metal nanoparticles (such as nanosized silver, iron, chromium, molybdenum, manganese, vanadium), low-valence reductive manganese or molybdenum compounds and the like, is used for prevention and treatment of tumors, especially skin tumors.

In an embodiment of the method for inhibiting glycolysis in cells according to the present invention, formic acid, formate, oxalic acid, oxalate or dioxalate is used as one of the ingredients in trace amount to moderate amount (e.g. a reducing agent at 0.001% to 5%) in pickles, pickles in soy sauce, dry goods, rice flour, rice flour products, flour, flour products, daily drinks, mineral water, bottled water or drinking water, for the purpose of prevention of tumors, especially tumors in the digestive tract, and antisepsis and preservation of food.

In an embodiment of the method for inhibiting glycolysis in cells according to the present invention, a reducing substance is used to prevent the hydrogen cycling process required for the redox reactions in glycolysis in anaerobia and facultative anaerobia, for preventing and treating bacterial infections, preservation of food, cosmetics of a natural origin, and perfumes, bactericidal protection for seeds and plants, and disease and infection prevention in local environment such as poultry farms. The reducing substance includes but is not limited to, selenium dioxide, selenite, formic acid, formate, oxalic acid, oxalate, dioxalate, sulfide (hydrogen sulfide, sodium sulfide, etc.), sulfurous acid, sulfite, sulfinic acid, sulfinate, dithionous acid, thiourea dioxide, thiocyanate, reductive amino acids, metal nanoparticles and nano-selenium, which may be used alone or in combination, for example, as a combinational formulation of oxalic acid with oxalate, a combinational formulation of formic acid with formate, or a combinational formulation of a solution, obtained by combining formic acid with formate, with acetic acid and/or ethanol. It is most preferably to use formate, oxalate, formic acid, oxalic acid and sodium selenite, which are non-toxic or less toxic.

The bacterial infections include, but are not limited to, inflammations by anaerobia or facultative anaerobia in the digestive tract, periodontitis, pharyngitis, oral inflammation, nasal inflammation, hircismus, human skin diseases such as onychomycosis, dermatophytosis, psoriasis, scalp mould, mycotic venereal diseases and bromhidrosis, and microbial infections in respiratory tract, digestive tract, urethra or lung. Most preferably, the reducing agent is used in the form of an oral agent, a spray, a fumigant, a lotion or mouthwash having water, an ethanol solution or an acetic acid solution as a carrier, or in the form of a paste or bath salt.

In an embodiment of the method for inhibiting glycolysis in cells according to the present invention, the antioxidant property and the anaerobic metabolism-inhibiting property of selenium dioxide, selenite, formic acid, formate, oxalic acid, oxalate and dioxalate are used for antisepsis, preservation, and anti-oxidation of food products including, but not limited to, fresh agricultural and fish products, rice flour, flour, fish, fruits and vegetables, natural flavors, and cosmetics including, but not limited to, cosmetics of a natural origin, detergent products, natural skin care products and daily cleaning and sanitary products. The use in the detergent products is to add the above reducing agent such as selenium dioxide, selenite or nano-selenium or formic acid, formate, oxalic acid, or oxalate in an aqueous solution, detergent, soap, toothpaste or mouthwash as a sterilizing agent for human body and environments such as kitchen or toilet for cleaning and sanitation.

Still another objective of the present invention is to provide antioxidant protection by using the antioxidant property of a reducing agent. That is, in an embodiment of the method for inhibiting glycolysis in cells according to the present invention, formic acid, formate, oxalic acid, oxalate, or dioxalate is used as a component or one of the components in a skin care product for antibacterial and antioxidant protection for human skin, by using the reducing substance to block the hydrogen cycling process required for the redox reactions in glycolysis in anaerobia and facultative anaerobia, and also using its antioxidant property.

In an embodiment of the method for inhibiting glycolysis in cells according to the present invention, reductive selenium dioxide, selenite, formic acid, formate, oxalic acid, oxalate, dioxalate or the like is used in the pre-immersion or washing process with water in food processing, to inhibit the metabolic process in anaerobia or facultative anaerobia, and to avoid or delay the acidification of water caused by fermentation.

Thus, in one aspect, the invention provides a method for inhibiting glycolysis in cells, comprising inhibiting glycolysis by using a reducing agent; preferably, the reducing agent is a small-molecule reducing agent. The small-molecule reducing agent in the present invention is different from a conventional small-molecule drug; the latter mainly refers to a chemically synthetic drug, particularly an organic compound having a molecular weight of less than 1,000, while the small-molecule reducing agent in the present invention can be just an ultra- or supra-small-molecule drug. Therefore, it has the same or greater advantages than traditional small-molecule drugs. For example, the most significant advantages of the small-molecule drug are low cost, low technical requirements for production, transportation and storage, and convenient for administration to patients (oral administration). In addition, the chemical small molecules can permeate the cell membranes and affect the expression and function of targets in cells, and thus have a wider application range. The small-molecule drugs are mostly absorbed as driven by a concentration gradient, without the aid of an active transporting system of proteins through epithelial cells in the digestive tract as required by macromolecular drugs or macromolecular substances. This has not only a quick effect and less loss along the absorption path, but also little side effects on the human body.

According to some embodiments of the present invention, in the method for inhibiting glycolysis in cells according to the present invention, the reducing agent is at a high concentration.

According to some embodiments of the present invention, in the method for inhibiting glycolysis in cells according to the present invention, a reducing agent is used to inhibit glycolysis in cells to treat a tumor disease.

According to some embodiments of the present invention, in the method for inhibiting glycolysis in cells according to the present invention, a reducing agent is used to inhibit glycolysis in cells to treat an anaerobic infection.

According to some embodiments of the present invention, in the method for inhibiting glycolysis in cells according to the present invention, the reducing agent is one or more selected from:
an organic reducing agent having not more than 5 carbon atoms, including but not limited to formic acid, formate, oxalic acid, oxalate, dioxalate, methionine, cysteine, thiourea dioxide, sulfinic acid, and sulfinate;
a sulfur-containing inorganic reducing agent having not more than 8 atoms, including but not limited to thiocyanide, thiosulfate, sodium sulfide, hydrogen sulfide, sulfurous acid, sulfite, and dithionite;
reductive metal nanoparticles or selenium nanoparticles, including but not limited to nanosized silver, iron, molybdenum, manganese, chromium, vanadium, and low-valence reductive manganese or molybdenum compounds.

More specifically, the reducing agent is one or more selected from reductive selenium dioxide, selenite, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, wherein the selenite, formate, oxalate, thiocyanate, thiosulfate and sulfite include but are not limited to salts of sodium, potassium, calcium, magnesium or ammonium.

According to some embodiments of the present invention, in the method for inhibiting glycolysis in cells according to the present invention, the process of inhibiting glycolysis in cells is to inhibit the hydrogen cycling process required for the redox reactions in glycolysis.

According to some embodiments of the present invention, in the method for inhibiting glycolysis in cells according to the present invention, the reducing agents include, but not limited to, formic acid, formate, oxalic acid, oxalate, dioxalate, reductive amino acid, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles, and nano selenium, used alone or in combination in a form of mouthwash or by oral administration, for prevention or treatment of tumors. It is most preferably to use formate, oxalate, formic acid, and/or oxalic acid, which are non-toxic or less toxic and not directly involved in biochemical reactions in cells.

According to some embodiments of the present invention, in the method for inhibiting glycolysis in cells according to the present invention, the reducing agent is one or more selected from reductive selenium dioxide, selenite, formic acid, formate, oxalic acid, oxalate, dioxalate, reductive amino acid, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, which are used in a form of an injection for transfusion, a paste for external application, spray, a stifling agent, bath salt or mouthwash, for prevention or treatment of tumors. It is most preferably to use formate, oxalate, formic acid, oxalic acid and/or sodium selenite, which are non-toxic or less toxic, as a reducing agent in a form of an injection for transfusion, a paste for external application, spray, a stifling agent, bath salt or mouthwash, for prevention or treatment of tumors. It is most preferably to use formate, oxalate, formic acid, oxalic acid and/or sodium selenite, which are non-toxic or less toxic.

According to some embodiments of the present invention, in the method for inhibiting glycolysis in cells according to the present invention, the reducing agent is one or more selected from: a low concentration of sulfinic acid, sulfinate, thiosulfate, thiocyanate, dithionous acid, thiourea dioxide, sulfides (hydrogen sulfide, sodium sulfide, etc.), a paste of sulfur powder, nano-sulfur powder or sulfur fine powder, low-toxic metal nanoparticles (such as nanosized silver, iron, chromium, molybdenum, manganese, or vanadium), low-valence reductive manganese or molybdenum compounds or the like, which is used for prevention and treatment of tumors, especially skin tumors.

According to some embodiments of the present invention, in the method for inhibiting glycolysis in cells according to the present invention,
the reducing agent is used in combination with traditional therapies such as surgery, radiotherapy, chemotherapy and targeted therapy, to help avoid tumor metastasis and recurrence; or
the reducing agent is used in combination with a traditional invasive inspection process such as centesis and microtomy to help avoid tumor metastasis and spreading; most preferably, the reducing agent is administered before the inspection.

According to some embodiments of the present invention, in the method for inhibiting glycolysis in cells according to the present invention, the reducing agent is used to block the hydrogen cycling process required for the redox reactions in glycolysis in anaerobia and facultative anaerobia, to achieve the effects and purposes of prevention and treatment of bacterial infections, preservation of food and cosmetics of a natural origin, sterilization protection for seeds and plants, and prevention of diseases and infections in poultry farms. The reducing agents include but are not limited to reductive selenium dioxide, selenite, formic acid, formate, oxalic acid, oxalate, dioxalate, reductive amino acid, thiourea dioxide, sulfinic acid, sulfinate, thiocyanide, thiosulfate, sulfide (sulfuric acid, sodium sulfite, etc.), sulfurous acid, sulfite, dithionous acid, metal nanoparticles, or nano selenium, used alone or in combination. It is most preferably to use formate, oxalate, formic acid, oxalic acid or sodium selenite, which are non-toxic or less toxic.

According to some embodiments of the present invention, in the method of inhibiting glycolysis in cells according to the present invention, the bacterial infection includes, but is not limited to, inflammations caused by anaerobia or facultative anaerobia in the digestive tract, periodontitis, pharyngitis, oral inflammation, nasal inflammation, hircismus, human skin diseases such as onychomycosis, dermatophytosis, psoriasis, scalp mould, mycotic venereal diseases and bromhidrosis, and microbial infections in the respiratory tract, digestive tract, urethra or lung. Most preferably, the reducing agent is used in the form of an oral agent, a spray, a fumigant, a lotion or mouthwash having water, an ethanol solution or an acetic acid solution as a carrier, or in the form of a paste or bath salt.

According to some embodiments of the present invention, in the method for inhibiting glycolysis in cells according to the present invention, the reducing agent such as sodium formate, sodium oxalate, potassium formate or potassium oxalate may produce, via glycolysis in cells or microorganisms in the intestinal tract or via free radical oxidation, alkaline sodium carbonate or potassium carbonate, which neutralizes acidic substances *in vivo,* such as uric acid, glutamic acid, and gastric acid, and promotes excretion of uric acid in the form of water-soluble sodium urate, so as to treat gout and/or reduce onset of gout, or reduce the diseases caused by acidic body fluids.

In another aspect, there is provided a use of a reducing agent in the preparation of a medicament for preventing and/or treating tumors by inhibiting glycolysis in cells, wherein the reducing agent includes, but is not limited to, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid and metal nanoparticles, used alone or in combination. It is most preferably to use formate, oxalate, formic acid, and/or oxalic acid, which are non-toxic or less toxic and not directly involved in biochemical reactions in cells.

In another aspect, there is provided a use of a reducing agent in the preparation of a medicament for preventing and/or treating anaerobia infections by inhibiting glycolysis in cells, wherein the reducing agent includes, but is not limited to, selenium dioxide, sodium selenite, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, used alone or in combination. It is most preferably to use formate, oxalate, formic acid, and/or oxalic acid, which are non-toxic or less toxic and not directly involved in biochemical reactions in cells.

In another aspect, there is provided a use of a reducing agent in the preparation of a medicament for treating gout and/or reducing the onset of gout, or reducing diseases caused by acidic body fluids, wherein the medicament may produce, via glycolysis metabolism in cells or microorganisms in the intestinal tract in human or via free radical oxidation in human, alkaline sodium carbonate, which neutralizes acidic substances *in vivo,* such as uric acid, glutamic acid, and gastric acid, and promotes excretion of uric acid in the form of water-soluble sodium urate, so as to treat gout and/or reduce the onset of gout, or reduce diseases caused by acidic body fluids, wherein the reducing agent includes sodium formate and sodium oxalate.

In another aspect, there is provided a use of a reducing agent in the preparation of a food product that prevents tumors and/or achieves food preservation by inhibiting glycolysis in cells, wherein formic acid, formate, oxalic acid, oxalate and/or dioxalate are used as one of the ingredients in trace amount to moderate amount in pickles, pickles in soy sauce, dry goods, rice flour, rice flour products, flour, flour products, daily drinks, mineral water, bottled water or drinking water.

In another aspect, there is provided a use of a reducing agent in the preparation of a food product which inhibits anaerobia metabolism by inhibiting glycolysis in cells, wherein the reducing agent includes selenium dioxide, selenite, formic acid, formate, oxalic acid, oxalate and/or dioxalate, and the food product includes, but is not limited to, fresh agricultural and fish products, rice flour, flour, fish, fruits and vegetables, and natural flavors.

In another aspect, there is provided a use of a reducing agent in the preparation of cosmetics which is capable of antisepsis, preservation and prevention of diseases by inhibiting glycolysis in cells, wherein the reducing agent includes selenium dioxide, selenite, formic acid, formate, oxalic acid, oxalate and/or dioxalate, and the cosmetics include, but are not limited to, detergents, skin care products, and personal daily cleaning and sanitary products.

In another aspect, there is provided a use of a reducing agent in the preparation of skin care products which provide anti-bacterial and antioxidation protection for human skin by inhibiting glycolysis in cells, wherein skin care products provide anti-bacterial and antioxidation protection on human skin by using the reducing agent's property of anti-oxidation and property of blocking the hydrogen cycling process required for the redox reactions in glycolysis in anaerobia and facultative anaerobia, wherein the reducing agent such as formic acid, formate, oxalic acid, oxalate or dioxalate is used as the ingredient or as one of the ingredients in the skin care product.

In another aspect, there is provided a use of a reducing agent in the preparation of a food product which avoids or delays the acidification of water caused by fermentation by inhibiting glycolysis in cells, wherein the reducing agent such as selenium dioxide, selenite, formic acid, formate, oxalic acid, oxalate or dioxalate is used in the pre-immersion or washing process with water in food processing, to inhibit the metabolic process in anaerobia or facultative anaerobia, so as to avoid or delay the acidification of water caused by fermentation.

### Detailed Description of Embodiments

### Example 1

To verify the inhibitory effect of the reducing agent on anaerobic fermentation, 1.5 g flour that had been exposed to the air and 10 ml tap water were mixed and poured into a transparent glass test tube with a diameter of 1.5 cm, stirred thoroughly every 10 minutes to make the paste fully exposed to air to promote microbial infection. After half an hour, 5 ml rapeseed oil was poured into the test tube to block the air. 20 samples were made, left stand for about 7 months from summer to winter for spontaneous fermentation, and observed for the final fermentation state.

To the 20 test tubes, before blocking the air with rapeseed oil, about 100 µg selenium dioxide, about 100 µg sodium selenite, about 1 ml formic acid, 0.1 g sodium formate, 0.1 g calcium formate, 0.1 g oxalic acid, 0.1 g sodium oxalate, 0.1 g sodium sulfide, 0.2 g L-cysteine (a sulfur-containing amino acid), 0.2 g sodium sulfate, 0.2 g glutamic acid, 2 ml ethanol, 2 ml acetic acid, 0.2 g sodium carbonate, 0.2 g ferrous sulfate, 0.2 g ferric sulfate, 0.2 g aluminum sulfate, 0.2 g calcium chloride, and 0.2 g sodium chloride were added respectively, leaving one test tube without such addition as a blank control.

After standing for about 7 months from summer to winter for spontaneous fermentation, it was finally found that, in the test tubes in which selenium dioxide, sodium selenite, formic acid, sodium formate, calcium formate, oxalic acid, sodium oxalate, sodium sulfide, L-cysteine and ferrous sulfate with an appropriate level of reductivity were added, the aqueous solution above the settled flour was clear and transparent, while in the other test tubes, in the aqueous solution above the flour there were milky white flocculated organic secretions with a various height of about 0.2 cm to 1.5 cm, which were from the metabolic anaerobic process of anaerobia, with the highest level of secretions of 1.5 cm in the blank control test tube. This result indicates that a reducing agent with an appropriate level of reductivity can inhibit the metabolic chemical reaction in anaerobia. Similarly, other sulfur-containing reducing agents such as sulfinic acid, sulfinate, dithionous acid, thiourea dioxide, α-thiopropionylglycine, lipoic acid, dihydrolipoic acid, disulfiram, thiol-containing compounds, disulfide-bond-containing compounds and the like also produced the same preventive and therapeutic effects on tumors and bacterial infections.

In order to further prove that the reducing agent only inhibits anaerobic glycolytic metabolism without affecting the aerobic metabolism, about 0.05 g of sodium selenite powder was further added to each of the blank control test tube as described above and the test tube containing sodium selenite. After about 6 hours (at a temperature of 20 to 38°C in summer), red products appeared in both test tubes, indicating that selenium in sodium selenite was reduced into red elemental selenium, distributed over the milky white secretions and above and within the flour paste, but no more secretions or new secretions appeared because the aerobic metabolites were carbon dioxide and water. Under an oil-sealed anoxic condition in which the anaerobic metabolism was suppressed by the reducing agent, sodium selenite provided oxygen for the aerobic metabolism in the bacteria, so that the aerobic reaction proceeded to reduce sodium selenite to elemental selenium and sodium salts of an organic acid. This also indicates that the presence of the reducing agent does not affect the aerobic metabolic process, but only inhibits the anaerobic glycolytic metabolic reactions. Apparently, the reducing agent only directly or indirectly inhibits the part of chemical reactions in the energy metabolism of the anaerobic and aerobic glycolysis that is different from the corresponding part in aerobic metabolism, i.e. the chemical reaction of hydrogenation of pyruvate, but does not affect the chemical reactions in the aerobic metabolism after glycolysis towards the tricarboxylic acid cycle. It also shows that the reductivity of the reducing agent does not interfere with metabolic reactions in normal cells, and thus has no toxicity to the aerobic metabolism in normal cells.

The chemical reactions involved in glycolysis in cells are invariable, independent of intracellular gene activity, cell morphology (for example, cancer cells in early, middle, and late stages, or recurrent or metastatic cancer cells, or cells at different stages of bacterial infection) or the location for cell growth (such as focus cells in different parts of the body and organs). As long as it is proven and confirmed to be able to affect, interfere with, or control a single step of the chemical reaction, the control of the metabolic process in cells is definite and not random, and the treatment effect thereof is not limited to a statistical meaning, but is definite.

### Example 2

In order to verify the preservative effect of the reducing agent on food, natural skin care products and the like, a lettuce leaf was symmetrically cut into two pieces having a size of 2 × 2 cm, which were placed respectively into two 50 ml open beakers with a diameter of 45 mm, each containing 80 g tap water, and sodium selenite having 180 µg selenium was added to one of the beakers. At room temperature of about 15 to 20°C, on the surface of the lettuce leaf in the sodium selenite-containing solution, a brown rot spot with a diameter of about 1 mm emerged on the fifth day, while on the lettuce leaf in the solution without sodium selenite, a round spot with a diameter of about 6 mm appeared. Comparative experiments using other plants, such as loofah juice which may be used for a skin care product, also showed significantly different rotting rates, that is, a bacterial spot with a diameter of up to about 3 mm was developed on the second day on the surface of the loofah juice without sodium selenite, while a spot did not appear on the surface of the loofah juice with sodium selenite until the 3rd day, and the spot further grew to a diameter of about 3 mm on the fifth day. In particular, a celery leaf in a solution free of sodium selenite turned yellow earlier than that in a solution having 0.5 ppm sodium selenite, while a celery leaf in a solution of sodium selenite did not fade or faded slowly, which indicates that the antioxidant property of sodium selenite provided an antioxidant protection for food.

Two portions of flour (50 g each) were used for fermentation experiments. Each portion was mixed with an equal amount (about 50 g) of water, and to one of the mixtures sodium selenite having about 200 µg selenium was added. After 2 days of fermentation (night temperature: about 15°C, and daytime temperature: about 35°C), the flour with sodium selenite still emitted a flour fragrance, while the flour without sodium selenite obviously emitted an acidic odor. The same phenomenon was observed over 20 days of continuous monitoring (the contrast between flour fragrance and the acidic odor). As measured by pH test paper, the flour with sodium selenite on the second day of fermentation was slightly alkaline with a pH of 7-8, while the flour without sodium selenite was acidified after fermentation with a pH of 4-5. This indicates that sodium selenite changed the metabolic pattern of the fermenting microorganism, forcing the microorganism to proliferate and grow via aerobic respiration in which the excreta was colorless and odorless carbon dioxide and water (carbon dioxide and water reacted with sodium selenite to produce alkaline sodium bicarbonate). Since tumor cells and anaerobia cells have the same steps and processes of chemical reactions in glycolysis, it is believed that tumor cells would either stop metabolism and die, or change the metabolic pattern and turn into normal cells.

Two pieces of meat slices of the similar size (about 1×2 cm in size, and about 1 mm in thickness) were used for a preservative experiment, and on one of them a sodium selenite tablet (containing about 100 µg selenium) was placed, with the other one remaining bare and free of sodium selenite. In order to make the sodium selenite infiltrate the meat slices, the same amount of water was sprayed onto both sides of the meat slice. In the evening of the same day, fly maggot eggs were found at only 5 positions on the meat slices on which sodium selenite was placed, while there were densely distributed maggot eggs on the bare meat free of sodium selenite. In the next morning, it was found that there was no maggot on the meat slice with sodium selenite, and the maggot eggs thereon had dried up, while a group of maggots appeared underneath the meat slice without sodium selenite. Because maggots can only grow on rot meat, and the meat slice with sodium selenite would not easily rot or can only be consumed by bacteria in an aerobic way that does not produce organic excretions, the maggot eggs cannot hatch and survive thereon. This experiment demonstrates that the reducing agent can be used not only for providing sterilization and preservative protection for vegetable food and seeds, and for prevention of diseases and infections in poultry farms, but also for antisepsis and preservation of protein-rich flour-made food and meats. In particular, use of formic acid or formate instead of formaldehyde as a preservative avoids the toxicity issue of formaldehyde.

The same phenomenon was observed when the above sodium selenite was replaced with selenium dioxide or formic acid, sodium formate or calcium formate, oxalic acid, sodium oxalate or a sulfur-containing reducing agent. According to the above experimental procedure for meat preservation, 0.01 g selenium dioxide powder, 1 ml formic acid, 0.1 g sodium formate powder, 0.1 g of calcium formate powder, 0.1 g oxalic acid powder, 0.1 g sodium oxalate powder, or 1 ml saturated solution of sodium sulfide was placed (and a small amount of water was sprayed after it was placed) or applied on the surface of one meat slice. The same result was obtained: there were very few maggot eggs (about 5) on the meat slice infiltrated with the reducing agent, and no maggot was hatched from these eggs. Therefore, it was verified and demonstrated that the inhibition on bacteria was due to the reducing effect of these agents, rather than due to the different elements in the substance *per se,* as the different substances were only carriers for the reducing effect. Because formic acid, oxalic acid, formate and oxalate have low toxicity, they may be used in an increased amount according to different requirements, such as the shelf life. They may be added to various food raw materials or processed food products in an amount of 0.01% to 5%.

Considering the cost, it is a better option to add a reducing agent (antioxidant) such as formic acid, formate, oxalic acid, or oxalate, instead of antioxidants such as vitamin C or vitamin E, in a meat product, to achieve the same effect of avoiding production of ammonium nitrite. It may also solve the toxicity issue of sodium nitrite, thiocyanate, and sulfite by replacing them with the reducing agent as a preservative for a meat product.

In summary, in the field of preventing decay caused by bacteria or microbes, that is, in the field of preservation, including antisepsis and preservation of food and cosmetics, the corresponding product forms may include various food preservatives, preservatives in the processing of fresh and cooked foods, and preservatives for natural flavors and cosmetic, etc.

### Example 3

In order to verify the therapeutic effect of a reducing agent such as sodium selenite on diseases such as skin diseases caused by bacteria, sodium selenite was added to 75% medical alcohol to prepare a solution of sodium selenite in alcohol containing 20 ppm selenium. The solution was applied once a day to a patient having been suffering from onychomycosis for 3 years. On the third day, the toenails were found to have changed from grayish yellow to a relatively normal color. Subsequently, the solution was applied to about 60 people suffering from onychomycosis, among whom one had onychomycosis for the longest period of 36 years. All of the patients had the infected nails exfoliated in one week to six months (the varying healing effect was due to the different frequency of application, as some patients forgot to apply the solution as long as he/she did not feel uncomfortable) and grew new healthy nails. This solution was applied once a day to the psoriasis or itchy skin that had lasted for 6 years, and the symptoms of itching and psoriasis basically disappeared after 2 weeks. After administration to about 100 people suffering from tineapediss or vesicles on feet, acne on the face, itching after mechanical cuts, or itching on skin with unknown reason for about half a year, all patients showed an improvement in skin appearance to a healthy state and itching relief.

A 5% acetic acid solution (analytically pure) was used to prepare a solution of sodium selenite in acetic acid containing 100 ppm selenium. This solution was applied to a patient having a history of hircismus, and the hircismus odor substantially disappeared on the same day. Then the solution was administrated to 16 patients with hircismus, and exactly the same effect was obtained without exception. Trials were also conducted on patients with gastric ulcer as confirmed by gastroscope. Sodium selenite having 91.3 µg selenium was supplemented to these patients every two days without other drugs. After 2 weeks, the stomach discomfort basically disappeared, indicating that the symptoms of gastric ulcer were improved, which may be because sodium selenite inhibited the harmful bacteria. In particular, among about 80 patients with tumors who took a reducing agent such as sodium formate, with about 30 also having gastrointestinal inflammation, all reported alleviated gastrointestinal discomfort.

Based on the bacteriocidal mechanism of a reducing agent discovered and verified in the present invention, a selenium-containing reducing agent is effective or assistantly effective in treatment of bacterial, mycotic, fungal rhinitis, or oral inflammation, pharyngitis, respiratory inflammation, pulmonary microbial infection, esophagus infection, gastrointestinal infection, urinary tract infection, blood infection, skin diseases and the like. It is especially effective in treatment of infections caused by antibiotic-resistant anaerobic or facultative anaerobic bacteria or aerobic superbacteria (because the reducing agent controls the metabolic reaction in glycolysis independent of the gene regulation or mutation). Similarly, the reducing agent is also effective as a bactericide for kitchens and bathrooms.

For the above-mentioned trials for treatment and prevention of skin diseases including skin tumors, a reducing agent such as selenium dioxide or formic acid, sodium formate, oxalic acid or sodium oxalate was used instead, and the same therapeutic effects were found. Among about 60 patients with onychomycosis mentioned above, about 35 patients who were not cured or not completely improved after 3 months of treatment with the above treatment were treated by spraying 5% acetic acid solutions each containing 0.01% of selenium dioxide, 1% of formic acid, 5% of sodium formate, 3% of oxalic acid, or 1% of sodium oxalate in a random manner, 150 ml each time. The patients may spray the solution every day or every few days on their own decisions. It was shown that the therapeutic effect was gradually increasing every day, and the visible onychomycosis disappeared after continuous treatment for 3 to 6 months.

However, it is found that it's better to increase the concentration of formic acid or sodium formate to 3% by weight to obtain a significant therapeutic effect for treatment of bacteria or microorganisms parasitic on skin, such as hircismus. Obviously, the higher the concentration of the reducing agent, the more excellent the bactericidal effect is, but an excessively high concentration would cause an uncomfortable salty feeling to skin.

For convenience in use, a selenium-containing reducing agent, or formic acid, formate, oxalic acid, oxalate, or a sulfur-containing reducing agent (bactericide) can be formulated into a spray or an aqueous solution, including a mouthwash, alcoholic agent, acetic agent, various pastes, bath salt or injection. Depending on specific characteristics of a specific disease and its specific focus, the concentration and dose of the reducing agent may be varied; for example, formic acid or formate can be used as a saturated solution. The content of excipient such as ethanol and acetic acid may also be varied, taking into account cost and odor comfort. Specifically, spraying on the surface of diseased skin can be performed with a large dose, while administration of a mouthwash is mainly based on the mouthfeel of different individuals. The dosage of oral agents or injections needs to be adjusted according to the health state and the functioning of the immune system of patients. A balance between the dose producing the fastest therapeutic effect and a dose tolerable by a patient's body may be gradually established by trying a small amount first and then increasing the does little by little, with reference to the personal comfort of the patient.

In addition, addition of a reducing agent such as sodium selenite to a cosmetic, a skin care product or a rubbing perfume may not only exploit its bactericidal effect but also utilize its antioxidant property to provide protection for human skin. In a practical trial, an aqueous solution of sodium selenite at a concentration of 5 ppm was sprayed on the brown spots on the face. After 2 weeks, the colored spots obviously faded, and after 2 months, the colored spots generally disappeared. More than 10 people used this solution, as well as randomly a 3% aqueous sodium formate solution, and all of them saw faded or disappeared colored spots.

Compared with selenite or selenium dioxide, formate and oxalate do not have the issue of toxicity or potential toxicity of selenium to a human body, and thus could be the best choices, and their amount added in food, cosmetics or skin care products may be increased as much as possible within the safe limit. For example, the toxic dose of selenium is about 400 mg per day, while formate may be taken by 1 to 5 g, which makes a thousand- or tens of thousand-time difference in the number of molecules. If natural skin-care raw materials or natural perfumes were dissolved and extracted with water, ethanol or acetic acid, such as loofah juice, aloe vera juice, wormwood, rose and various wild-weed fragrance, and a reducing agent such as formate or oxalate was added thereto, then the bacteriocidal effect and skin protecting effect of the product were further improved.

### Example 4

A patient with advanced malignant tongue root tumor had been provided with only painkillers by a hospital because of lymphatic metastasis and the age of 80. This patient was then given the combination of reducing agents in Example 1 in cycles (the daily dosing regime was 1 g sodium formate, 0.5 g sodium oxalate and 0.5 g cysteine for the morning, noon, and evening, respectively; after 10 days, the regime was changed to 1.5 g calcium formate, 0.5 g oxalic acid and 400 µg sodium selenite for the morning, noon, and evening, respectively; after another 10 days, the regime was changed to 15 ml of 5% formic acid, 0.1 g sodium sulfide, and 400 µg sodium selenite for the morning, noon, and evening, respectively; and the cycle was repeated). After two and a half months, the facial lymph lump was reduced from about 6 cm in diameter to be invisible to naked eyes, and the tongue root lump also disappeared to be invisible. The advantage of the cyclic medication is that it can reduce or avoid in advance the possibility of drug resistance development by tumor cells, and the same reason also applies below. However, in order to reduce the complexity during administration, one or a few of the above reducing agents may be taken, in which case sodium formate or calcium formate is preferred, while sodium oxalate or oxalic acid or formic acid could be the second choice.

A 45-year-old patient had a subaxillary lymphoid lump of about 8 cm and a red thyrsoid lump on the chest skin covering an area of 3 × 3 cm due to metastatis from the primary site lung. This patient was given the above composition by oral administration everyday in cycles, and also directly rubbed the above reducing agents onto skin 3 to 6 times or randomly sprayed a 0.5% ferrous sulfate solution or a 0.05% sodium sulfide solution. After the above combination of reducing agents was applied for about 2 months, the subaxillary lymphoid lump basically disappeared, and the red carcinoma (tubercule) on skin was mostly detached after calcification into a white scab, with a dark residue still remaining. When the white scab on skin was only about 2 cm in diameter, the patient actively removed some scabs on the surface during washing, resulting in a burst of growth of the skin lump on the next day, and after about 48 hours, the red skin lump increased to an area of about 20 × 20 cm, almost covering the entire right chest. This phenomenon directly demonstrates that explosive growth of cancer tumor would occur after it receives a mechanical stimulation. Thereafter, the above reducing agents were applied in cycles every hour (excluding nighttime), and on the next day the increase in the skin lump was controlled, and the red color of the lump faded and turned dark, indicating that the tumor lump was effectively controlled. After about a month of continuous application, most of the skin lump was calcified completely to form a scab. Therefore, it can be inferred that, in order to prevent the spreading and metastasis of cancer tumor tissue, when a patient is subjected to an invasive examination such as centesis or microtomy, a non-toxic and harmless reducing agent can be applied to provide preventive treatment for the patient, which is helpful to avoid spreading and metastasis of tumor.

A female patient with lung cancer had bone metastasis, and was given the reducing agents according to the above method for two months. Her previous backache disappeared. The CT inspection revealed that one lump among the three bone metastases had disappeared, and the other two focuses were also reduced in size.

A 55-year-old patient of a primary liver malignancy was treated with the above-mentioned cyclic method for 9 days. The CT inspection revealed that the lump was reduced from 8.7 cm to less than 8 cm.

A patient with laryngeal cancer who had been unable to eat or drink for about one month due to blockage of the esophagus was administered with the above combinational medicament in cycles every other hour when in consciousness by holding the medicament in mouth for a few minutes. After only one day the patient could drink a little water. The other two patients with advanced esophageal cancer and having a systemic pain were administrated with the above reducing agent for one day, and on the next day had their pain relieved and appetite increased. A patient with small cell lung cancer neck metastasis, having only 0.7 to 0.8 mm wide veins as shown by CT because the lump pressed against the vein at the neck, causing severe neck swelling and affecting normal breathing, was administrated with the above reducing agent. On the next day, the symptom of breathing difficulty was alleviated, and 8 days later CT inspection showed that the vein width increased to approximately 1.8 mm.

A patient with brain tumor was administrated according to the above-mentioned cyclic method for 20 days. Nuclear magnetic resonance showed that the size of the intracranial lump was reduced from 2.8 cm to about 1 cm, indicating that the small-molecule reducing agent can cross the blood-brain barrier.

A patient with advanced lung cancer showed a CY211 index of up to 28 during a 15-month course with bevacizumab as a targeted drug and remaining at about 15 to 16 later, and CT showed that the size of the lump maintained at about 1 × 2 cm. The patient was administrated with the reducing agents of the present invention according to the above-mentioned cyclic method for 63 days. Thereafter, the lump started to show thyrsoid splitting from inside, and the CY211 index was also reduced to a minimum value of 11. After 90 days, CT inspection confirmed that the tissue structure changed continuously in the lump, and the highlight part in the lump contracted to about 1 × 1.5 cm. The patient had a better body feeling than before during the administration, with little chest tightness or coughing. The menstruation that had stopped for nearly 2 years appeared again normally for the first time.

As a comparison, a patient diagnosed with hepatic cysts by color ultrasound in 2012 had not been treated since then. From June 2017, the patient was administrated with about 200 µg sodium selenite every day. The maximum cross-sectional areas of the hepatic cysts obtained from the patient's annual physical examination reports were plotted over time for analysis, which showed that the size of the hepatic cysts increased every year following a curve of upward parabola, but in the physical examination report in March 2018 the increase in hepatic cysts deviated from the parabolic rapid growth pattern and entered a low-speed growth pattern, i.e., a noticeably slowed-down growth rate. This indicates that sodium selenite has an effect of inhibiting lump growth, but it cannot realize the purpose of regressing and eliminating the tumor due to the limitation of safe dosage.

There are other reports of gradual improvements after the treatment, currently including nearly 80 patients in total, having brain tumors, laryngeal cancer, esophageal cancer, lung cancer, thyroid cancer, bone cancer, liver cancer, melanoma, breast cancer, uterine cancer, lymphoma, skin cancer or the like. The treatment process will not be specified here. During the treatment course, it was found that patients with advanced cancer needed to increase the dose to control the tumor growth when they felt progressed symptoms of cancer (such as increased pain). However, local swellings like small cob loafs often occurred on some positions on the patient's body (such as forearms, breast at chest), in which case reducing the dose of the reducing agent would make the swellings regress on the same day, while increasing the dose of the reducing agent would make the swellings enlarge. Therefore, although the amount of reducing agents should be increased as much as possible in order to cure tumors and cancer as soon as possible, the maximum dose of reducing agent should abide by the principle that the body does not show local swellings. Generally, the dose of each reducing agent may be adjusted at a high daily dose from 400 mg to 5 g. The reason is that the reducing agent inhibits glycolysis and the chemical reaction of glutamine metabolism in tumor cells, leading to disability and apoptosis of tumor cells, while the apoptotic tumor cells or cell debris need to be processed by the human lymphatic immune system. The tumor cell debris will accumulate in the lymph nodes due to the limitation of the immune treatment speed of the lymphatic system, causing lymphatic blockage. At this time, some places in the body may swell due to the blockage of lymphatic vessels.

The chemical reactions involved in glycolysis in cells are invariable, independent of intracellular gene activity, cell morphology (for example, cancer cells in early, middle, and late stages, or recurrent or metastatic cancer cells, or cells at different stages of bacterial infection) or the location for cell growth (such as focus cells in different parts of the body and organs). As long as it is proven and confirmed to be able to affect, interfere with, or control a single step of the chemical reaction, the control of the metabolic process in cells is definite and not random, and the treatment effect thereof is not limited to a statistical meaning, but is definite. However, it is necessary to further confirm whether tumor tissue or tumor cells can develope a mechanism or protective layer that blocks the entry of the reducing agent. Currently, a patient who had a tumor lump on the laryngeal node having an inflamed and ulcerated incision due to a surgical misoperation (misdiagnosed as abscess, but actually metastasized tumor tissue) was administrated with the above reducing agent according to the above-mentioned cyclic method for nearly 4 months. The wound lump tissue appeared to increase slowly, although the lumps that metastasized or recurred into the lung were no longer detected. It seems that the reducing agent wes hampered from entering cells of the tumor tissue because the tumor tissue was protected by secretions of bacteria in the wound (the infecting bacteria developed resistance to drugs except vancomycin). Therefore, it is a meaningful direction to further study how to efficiently deliver the reducing agents to the inside of tumor cells.

For skin cancer, it is more effective to directly apply the above reducing agent. In particular, a reducing agent at a low concentration (0.1% to 0.5%) may be used cyclically, such as sulfinic acid, sulfinate, dithionous acid, thiourea dioxide, sodium thiosulfate, thiocyanate, hydrogen sulfide, sodium sulfide, sulfide, a paste of sulfur powder, nano sulfur powder or sulfur fine powder, low-toxicity metal nanoparticles such as nanosized silver, iron, chromium, molybdenum, manganese or vanadium, and low-valence reductive manganese or molybdenum compounds.

In order to prevent various tumor diseases, especially inflammation or tumors in the digestive tract, non-toxic, harmless or low-toxic reducing agents such as formic acid, formate, oxalic acid and oxalate may be used as one of ingredients in trace amount to moderate amount in daily beverages, mineral water or bottled water or drinking water (such as 0.01% to 5%; for public products, the specific type and content of the reducing agents to be added should conform to the provisions under the National Food Safety Law) and produced into a special product in forms of beverages, or as one of ingredients in trace amount to moderate amount in dry products such as pickles, pickles in soy sauce, fruits and vegetables, rice flour, rice flour products, flour, flour products (including biscuits, snacks, etc.) in forms of food or mouthwash, to widely suppress gastrosis, gastritis, and inflammation and tumorigenesis in digestive tract and oral cavity. Unlike the use of formic acid as traditional acidifiers for mouthfeel, formic acid or formate acts to prevent inflammation and cytopathy in the present invention.

In addition, stem cells also gain energy mainly by glycolysis, while the direct involvement of a reducing agent in the redox reaction cycle intrinsic to glycolysis in cells is not controlled by chemical or biochemical reactions associated with intracellular gene activities or intracellular proteins and enzymes, and thus all reducing agents with sufficient reductivity have the same effect on the progress of the redox reactions in cells, that is, the chemical reactions between substances are only controlled by the chemical driving energy of the chemical reaction equations, not depending on the time and place. Therefore, similarly, the reducing agent can also inhibit the growth of stem cells and promote their apoptosis. It is known that traditional methods for treating tumors, such as radiotherapy, chemotherapy, and targeted therapy, may result in tumor cells turning towards stem cells, and these turned tumor cells are an important mechanism or one of the important mechanisms of cancer recurrence and metastasis. Therefore, a reducing agent in combination with conventional therapeutic methods such as radiotherapy, chemotherapy and targeted therapy would help avoid tumor metastasis and recurrence. At present, nearly 80 patients with different tumors who were administrated with reducing agents all reported that the lumps visibly regressed, or decreased in numbers. No new metastatic lump was reported. In addition, 3 patients with lung cancer, 2 with gastric cancer and one with breast cancer all reported that, during the course of chemotherapy, the side effects after chemotherapy were significantly weakened after taking the reducing agents of the present invention at a separate time, as compared with the cases in which the reducing agents were not administrated.

### Example 5

Considering that sodium formate and sodium oxalate may produce, via glycolysis metabolism in cells or microorganisms in the intestinal tract or via free radical oxidation, alkaline sodium carbonate, then sodium formate, sodium oxalate, potassium formate or potassium oxalate may be given to indirectly increase alkalinity and neutralize acidic substances *in vivo,* such as uric acid, glutamic acid and stomach acid, so as to increase alkalinity of urine and promote excretion of uric acid in the form of water-soluble sodium urate, thereby realizing the effect of treating gout or reducing gout onset. Compared with the existing conventional method of administrating sodium bicarbonate or sodium carbonate to alleviate or solve the gout problem, sodium formate, sodium oxalate and the corresponding potassium salts do not have the issue of sodium bicarbonate of being easily neutralized by gastric acid to lose alkalinity and not easily effectively increasing the alkalinity in body fluids including blood

The trials on 2 patients with gout showed that the urine tested in the morning with test paper showed a pH of 5.5 to 6 during the gout onset, while after administration of sodium formate 3 times per day, 1 g each time, with a glass of water for convenient administration (the amount of water is not relevant to the therapeutic effect), continuously for 3 days, the pain feeling of gout was alleviated and even disappeared, and at this time, the urine tested in the morning with test paper showed an increased pH of 6 to 6.5. One of the patients experienced gout recurrence after eating wild vegetables. The patient was administrated with sodium oxalate instead, 1 g per day, and after 3 days, the pain feeling of gout disappeared, and the urine tested with test paper showed a pH of 7. Another patient with a tumor was administrated with sodium formate, 1 g per day, for two weeks. After that, the urine test in a hospital showed that the urine pH reached 8. This showed that sodium formate took effect as expected in theory and sodium urate in urine caused an increase in alkalinity of urine. Therefore, the administration of sodium formate can effectively increase alkalinity of body fluids, including blood, improve uric acid excretion *in vivo,* and thus prevent and treat diseases associated with high uric acid and acidic body fluids.

At present, 6 patients with frequent gout had finished the trial. Among them, 3 patients were administrated with sodium oxalate, 1 g per day. All of them showed an increased pH in the urine tested first in the morning, and the gout symptoms usually disappeared within 2 to 5 days.

### Example 6

In addition to glycolysis, tumor cells can also produce energy and nutrients by conversion of glutamine into α-ketoglutaric acid followed by the tricarboxylic acid cycle pathway, and have a metabolism via the pentose phosphate pathway to primarily produce nutrients rather than energy. Therefore, only blockage of both the glycolysis and the carboxylic acid cycle of glutamine can completely block the process of energy production in tumor cells. The pentose phosphate pathway would be meaningless if the cells are deprived of energy.

The chemical reactions involved in glutamine metabolism are as follows:
1. Glutamine is hydrolyzed under the catalysis by glutaminase to release alkaline ammonia, and converted to L-glutamate, while the ammonia is converted to urea in liver.
2. L-glutamate is dehydrogenated and oxidized by glutamate dehydrogenase and coenzyme NADP⁺ to form a glutamate-dehydrogenated intermediate, while NADP⁺ is reduced to NADPH; this step is a redox reaction.
3. The glutamate-dehydrogenated intermediate reacts with water to release ammonia again to form α-ketoglutaric acid.
4. α-ketoglutaric acid enters the tricarboxylic acid cycle.

Steps 2 and 3 above are reversible reactions, and generally favor the synthesis of glutamate. This indicates that the chemical driving force from glutamate to the dehydrogenated intermediate and to α-ketoglutaric acid is not strong and is susceptible to external competitive factors.

In order to prove the above assumption, 20 g flour paste (having a water content of about 60%) was thoroughly mixed with 15 g glutamine, and divided into 2 equal samples, each was placed in a 50 ml beaker, and to one beaker 0.1 mg of the reducing agent sodium selenite was added and stirred well. At room temperature of about 25°C, after one hour of spontaneous fermentation, the pH value of the gas in the beaker was measured with wet pH paper. It was visually confirmed that the beaker with sodium selenite had a pH value of about 7.5, and the beaker without sodium selenite had a pH value in the range of 8 to 9. It can be seen that the two beakers had alkalinities significantly different from each other, indicating that sodium selenite had an impact. That is, the reducing agent suppressed the above-mentioned redox reaction in Step 2 above, and prevented the reaction in Step 3, thereby reducing the production of ammonia in Step 3. In addition, as the fermentation proceeded, the flour paste without sodium selenite emitted an unpleasant odor, which was due to the odorous organic matter produced by anaerobic fermentation, while the flour paste with sodium selenite only produced a large amount of carbon dioxide bubbles formed by aerobic fermentation.

In order to confirm the above assumption, the above-mentioned glutamine was replaced with L-glutamate, and after spontaneous fermentation for one hour, the pH value of the gas in the beaker was measured with wet pH paper. It was visually confirmed that the beaker with sodium selenite had a pH value of about 7.5, and the beaker without sodium selenite had a pH value of 8. It can be seen that the two beakers had alkalinities significantly different from each other, indicating that not only the hydrolysis of glutamine released ammonia, but also the hydrolysis of L-glutamate after dehydrogenation also released ammonia. The difference in alkalinities indicates that L-glutamate cannot be effectively dehydrogenated in the presence of a reducing agent, and thus no subsequent reaction can proceed to release ammonia after adding water. In addition, dry test paper was inserted upright into the flour paste for 30 minutes. Obviously, the water absorbed on the test paper for the flour paste with sodium selenite reached a higher level than the flour paste without sodium selenite, at levels of 9 mm and 5 mm, respectively, which indicates that the flour paste with sodium selenite had a higher water content, while the flour paste without sodium selenite had a lower water content because L-glutamate can be dehydrogenated in the absence of a reducing agent and its dehydrogenated product may be converted to α-ketoglutaric acid by water absorption (Step 3).

According to the experimental procedure above, sodium selenite was replaced with 1 mg of selenium dioxide powder, 0.1 g of sodium formate powder, 0.1 g of calcium formate powder, 0.1 g of oxalic acid powder, or 0.1 g of sodium oxalate powder. The same results were obtained as follows: when the flour paste contained glutamine, the pH in the beaker with a reducing agent had a pH of about 7.5, while the beaker without the reducing agent had a pH in a range of 8 to 9; when the flour paste contained L-glutamate instead, the water absorbed on the test paper for the flour paste with a reducing agent reached a higher level than the flour paste without the reducing agent, at levels of about 9 mm and about 5 mm, respectively.

The principle of the above-mentioned reducing agents inhibiting glutamine metabolism is that the reducing agent reacts with water to generate a free hydrogen proton, which preferentially reacts with coenzyme NADP⁺ to form coenzyme NADPH, thereby blocking the dehydrogenation reaction of L-glutamate in Step 2. This example demonstrates that a reducing agent can act as a drug for inhibiting glutamine metabolism. The drugs may be used in forms of spray, pill, injection for transfusion or intervention, to prevent or directly treat tumors (because glutamine metabolism is one of the metabolism pathways in tumor cells other than the glucose glycolysis) or diseases associated with abnormal glutamine metabolism, including infectious diseases caused by microorganisms. It is also demonstrated that a reducing agent may inhibit both the glutamine metabolism and glycolysis (which is directly or indirectly demonstrated in Examples 1 to 3 of the present invention).

The inhibition of glutamine metabolism by the above reducing agent is a characteristic of pure chemical reaction in cells, especially the direct involvement in the intrinsic redox reactions in cells, and not chemical or biochemical reactions associated with intracellular gene actions or intracellular proteins, enzymes, etc. Therefore, all reducing agents with sufficient reductivity have the same inhibitory effect on glutamine metabolism. That is, the chemical reactions inherent between substances do not vary depending on the time and place. Similarly, seen from the results of the above examples, the reducing agents that can be used also include selenium dioxide, sodium selenite, formic acid, formate, oxalic acid, oxalate, dioxalate, sulfide, sulfurous acid, sulfite, sulfinic acid, sulfinate, thiocyanate, thiosulfate, dithionous acid, thiourea dioxide, reductive amino acids, nano-selenium, metal nanoparticles, low-toxic metal nanoparticles, such as nanosized silver, iron, molybdenum, manganese, chromium, vanadium, and low-valence reductive manganese and molybdenum compounds.

The chemical reactions involved in glutamine metabolism in cells are also invariable, independent of intracellular gene activity, cell morphology (for example, cancer cells in early, middle, and late stages, or recurrent or metastatic cancer cells) or the location for cell growth (such as focus cells in different parts of the body and organs). As long as it is proven and confirmed to be able to affect, interfere with, or control a single step of the chemical reaction, the control of the metabolic process in cells is definite and not random, and the treatment effect thereof is not limited to a statistical meaning, but is definite.

It can be concluded based on the glycolysis process, Example 1 and this example that the coenzymes NAD⁺ and NADP⁺ are the two most suitable targets for treatment of tumors. The reducing agent acts on these two targets to prevent the sustained reactions of glycolysis and glutamine metabolism in cells.

### Example 7

In the food industry, it is often necessary to soak or clean the processed products with water in advance, during which the food and water may become acidic due to microbial fermentation, thereby affecting the quality and taste of the processed food. In this example, reductive selenium dioxide, selenite, formic acid, formate, oxalic acid or oxalate was used in food processing, especially in the immersion procedure of glutinous rice in the processing of glutinous rice flour, to inhibit metabolism in anaerobic bacteria or facultative anaerobic bacteria and avoid or delay the acidification of water caused by fermentation.

In experiments for comparison, two portions of 50 g glutinous rice granules were taken, to each of them 100 ml tap water was added, and one of them contained 15 mg/L sodium formate. At room temperature of about 25°C, after 4 hours, the pH value of water in the glutinous rice without sodium formate decreased from about 6 to about 3, while the pH value of water in the glutinous rice with sodium formate only decreased from about 7 to about 6. When the concentration of sodium formate was 0.1 g/L, after 5.5 hours, the pH of water in the glutinous rice without sodium formate decreased from about 6 to about 3, while the pH of water in the glutinous rice with sodium formate slightly decreased from about 7. The same effect was obtained after replacing sodium formate with calcium formate.

As described in the above examples, by exploiting the same chemical reaction process between the anaerobic glycolysis in microorganisms and the aerobic glycolysis process in tumors, the consistent inhibitory effect of the reducing agent on the glycolysis process was mutually verified in above examples for microbial infection and the examples for treatment of tumor diseases.

The above description only addresses some preferred embodiments of the present invention, and anyone ordinarily skilled in the art can conceive of equivalent embodiments having partial change or modification by using the technical content disclosed in the present invention without departing from the technical scope of the present invention, and they are still within the scope of the technical scope of the invention.

## Claims

1. A method for inhibiting glycolysis in cells, comprising using a reducing agent to inhibit glycolysis in cells; preferably, the reducing agent is a small-molecule reducing agent.

2. The method according to claim 1, wherein the reducing agent is used at a high concentration.

3. The method according to claim 1 or 2, wherein the reducing agent is used to inhibit glycolysis in cells, so as to treat tumor diseases.

4. The method according to claim 1 or 2, wherein the reducing agent is used to inhibit glycolysis in cells, so as to treat anaerobia infections.

5. The method according to claim 1, wherein the reducing agent is one or more selected from:
an organic reducing agent having not more than 5 carbon atoms, including but not limited to formic acid, formate, oxalic acid, oxalate, dioxalate, methionine, cysteine, thiourea dioxide, sulfinic acid and sulfinate;
a sulfur-containing inorganic reducing agent having not more than 8 atoms, including but not limited to thiocyanide, thiosulfate, sodium sulfide, hydrogen sulfide, sulfurous acid, sulfite, and dithionous acid; and
reductive metal nanoparticles or selenium nanoparticles, including but not limited to nanosized silver, iron, molybdenum, manganese, chromium, vanadium, and low-valence reductive manganese or molybdenum compounds;
more specifically, the reducing agent is one or more selected from reductive selenium dioxide, selenite, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, wherein the selenite, formate, oxalate, thiocyanate, thiosulfate and sulfite include but are not limited to salts of sodium, potassium, calcium, magnesium or ammonium; and
the inhibiting glycolysis in cells is to suppress the hydrogen cycling process required for the redox reactions in glycolysis.

6. The method according to claim 1 or 3, wherein the reducing agent includes, but is not limited to, formic acid, formate, oxalic acid, oxalate, dioxalate, reductive amino acid, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles, nano selenium, used alone or in combination in a form of mouthwash or by oral administration, which are used for prevention or treatment of tumors; most preferably, formate, oxalate, formic acid, and/or oxalic acid which are non-toxic or less toxic and not directly involved in biochemical reactions in cells are used.

7. The method according to claim 1 or 3, wherein the reducing agent is one or more selected from reductive selenium dioxide, selenite, formic acid, formate, oxalic acid, oxalate, dioxalate, reductive amino acid, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, which are used in forms of injection for transfusion, paste for external application, spray, stifling agents, bath salt or mouthwash, for prevention or treatment of tumors; most preferably, formate, oxalate, formic acid, oxalic acid and/or sodium selenite which are non-toxic or less toxic are used.

8. The method according to claim 1 or 3, wherein the reducing agent is one or more selected from a low concentration of sulfinic acid, sulfinate, thiosulfate, thiocyanate, dithionous acid, thiourea dioxide, sulfides (hydrogen sulfide, sodium sulfide, etc.), pastes of sulfur powder, nano-sulfur powder and sulfur fine powder, low-toxic metal nanoparticles (such as nanosized silver, iron, chromium, molybdenum, manganese, vanadium), low-valence reductive manganese or molybdenum compounds, and the like, which are used for prevention and treatment of tumors, especially skin tumors.

9. The method according to claim 1 or 3, wherein the reducing agent is used in combination with traditional therapies such as surgery, radiotherapy, chemotherapy and targeted therapy, to help avoid tumor metastasis and recurrence; or
the reducing agent is used in combination with a traditional invasive inspection process such as centesis and microtomy to help avoid tumor metastasis and spreading; most preferably, the reducing agent is administered before the inspection.

10. The method according to claim 1 or 4, wherein the reducing agent is used to block the hydrogen cycling process required for the redox reactions in glycolysis in anaerobia and facultative anaerobia, to achieve the effects and purposes of prevention and treatment of bacterial infection, preservation of food and cosmetics of a natural origin, sterilization protection for seeds and plants, and prevention of diseases and infections in poultry farms, wherein the reducing agent includes but is not limited to reductive selenium dioxide, selenite, formic acid, formate, oxalic acid, oxalate, dioxalate, reductive amino acid, thiourea dioxide, sulfinic acid, sulfinate, thiocyanide, thiosulfate, sulfide (sulfuric acid, sodium sulfite, etc.), sulfurous acid, sulfite, dithionous acid, metal nanoparticles, and nano selenium, used alone or in combination; it is most preferably to use formate, oxalate, formic acid, oxalic acid or sodium selenite which are non-toxic or less toxic.

11. The method according to claim 10, wherein the bacterial infection includes, but is not limited to, inflammations caused by anaerobia or facultative anaerobia in the digestive tract, periodontitis, pharyngitis, oral inflammation, nasal inflammation, hircismus, human skin diseases such as onychomycosis, dermatophytosis, psoriasis, scalp mould, mycotic venereal diseases and bromhidrosis, and microbial infections in the respiratory tract, digestive tract, urethra or lung; most preferably, the reducing agent is used in the form of an oral agent, a spray, a fumigant, a lotion or mouthwash having water, an ethanol solution or an acetic acid solution as a carrier, or in the form of a paste or bath salt.

12. Use of a reducing agent in the preparation of a medicament for preventing and/or treating tumors by inhibiting glycolysis in cells, wherein the reducing agent includes, but is not limited to, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid and metal nanoparticles, used alone or in combination; it is most preferably to use formate, oxalate, formic acid, and/or oxalic acid, which are non-toxic or less toxic and not directly involved in biochemical reactions in cells.

13. Use of a reducing agent in the preparation of a medicament for preventing and/or treating anaerobia infections by inhibiting glycolysis in cells, wherein the reducing agent includes, but is not limited to, selenium dioxide, sodium selenite, formic acid, formate, oxalic acid, oxalate, dioxalate, reductive amino acids, thiourea dioxide, sulfinic acid, sulfinate, thiocyanide, thiosulfate, sulfides (hydrogen sulfide, sodium sulfide, etc.), sulfurous acid, sulfite, dithionous acid, metal nanoparticles and nano selenium, used alone or in combination; it is most preferably to use formate, oxalate, formic acid, and/or oxalic acid, which are non-toxic or less toxic and not directly involved in biochemical reactions in cells.

14. Use of a reducing agent in the preparation of a medicament for treating gout and/or reducing the onset of gout, or reducing diseases caused by acidic body fluids, wherein the medicament produces, via glycolysis metabolism in cells or microorganisms in the intestinal tract in a human body or via free radical oxidation in a human body, alkaline sodium carbonate or potassium carbonate, which neutralizes acidic substances *in vivo,* such as uric acid, glutamic acid, and gastric acid, and promotes excretion of uric acid in the form of water-soluble sodium urate, so as to treat gout and/or reduce the onset of gout, or reduce diseases caused by acidic body fluids, wherein the reducing agent includes sodium formate, sodium oxalate, potassium formate, or potassium oxalate.

15. Use of a reducing agent in the preparation of a food product that prevents tumors and/or achieves food preservation by inhibiting glycolysis in cells, wherein formic acid, formate, oxalic acid, oxalate and/or dioxalate are used as one of the ingredients in trace amount to moderate amount in pickles, pickles in soy sauce, dry goods, rice flour, rice flour products, flour, flour products, daily drinks, mineral water, bottled water or drinking water.

16. Use of a reducing agent in the preparation of a food product which inhibits anaerobia metabolism by inhibiting glycolysis in cells, wherein the reducing agent includes selenium dioxide, selenite, formic acid, formate, oxalic acid, oxalate and/or dioxalate, and the food product includes, but is not limited to, fresh agricultural and fish products, rice flour, flour, fish, fruits and vegetables, and natural flavors.

17. Use of a reducing agent in the preparation of cosmetics which is capable of antisepsis, preservation and prevention of diseases by inhibiting glycolysis in cells, wherein the reducing agent includes selenium dioxide, selenite, formic acid, formate, oxalic acid, oxalate and/or dioxalate, and the cosmetics include, but are not limited to, detergents, skin care products, and personal daily cleaning and sanitary products.

18. Use of a reducing agent in the preparation of a skin care product which provide anti-bacterial and anti-oxidation protection for human skin by inhibiting glycolysis in cells, wherein the skin care products provide anti-bacterial and anti-oxidation protection for human skin by using the reducing agent's property of anti-oxidation and property of blocking the hydrogen cycling process required for the redox reactions in glycolysis in anaerobia and facultative anaerobia, wherein the reducing agent formic acid, formate, oxalic acid, oxalate or dioxalate is used as the ingredient or as one of the ingredients in the skin care products.

19. Use of a reducing agent in the preparation of a food product which avoids or delays the acidification of water caused by fermentation by inhibiting glycolysis in cells, wherein reductive selenium dioxide, selenite, formic acid, formate, oxalic acid, oxalate or dioxalate is used in the pre-immersion or washing process with water in food processing, to inhibit the metabolic process in anaerobia or facultative anaerobia, so as to avoid or delay the acidification of water caused by fermentation.
